# DEMANDE DE BREVET EUROPEEN

(11) **EP 1 321 126 A1**
(43) Date de publication de la demande: **25.06.2003**
(21) Numéro de dépôt: 02292958.2
(22) Date de dépôt: 29.11.2002
(51) Int. Cl.: A61K 7/06

(54) **Compositions cosmétiques comprenant des polymères à fonctions chimiques complémentaires**

(30) Priorité: 18.12.2001 FR 0116387
(71) Demandeur: L'Oreal, 92585 Clichy Cédex (FR)
(72) Inventeur: Samain, Henri, 91570 Bievres (FR); Rollat-Corvol, Isabelle, 75017 Paris (FR); Vic, Gabin, 60280 Venette (FR); Livoreil, Aude, 75006 Paris (FR)
(74) Mandataire: Bourdeau, Françoise

(57) **Abrégé**

L'invention a pour objet des compositions cosmétiques, comprenant au moins deux polymères comprenant des fonctions chimiques complémentaires, aptes à former un gainage sur les matières kératiniques et en particulier sur les cheveux. Elle vise également un procédé cosmétique comprenant la mise en oeuvre de ces polymères sur les matières kératiniques, et en particulier sur les cheveux ainsi que leur utilisation pour réaliser un gainage sur les cheveux.

## Description

L'invention a pour objet des compositions cosmétiques, comprenant au moins deux polymères comprenant des fonctions chimiques complémentaires, aptes à former un gainage sur les matières kératiniques et en particulier sur les cheveux. Elle vise également un procédé cosmétique comprenant la mise en oeuvre de ces polymères sur les matières kératiniques, et en particulier sur les cheveux ainsi que leur utilisation pour réaliser un gainage sur les cheveux.

Les produits cosmétiques destinés aux traitements des cheveux emploient souvent des polymères. Ils permettent d'obtenir des effets de maintien de la forme de la coiffure, des effets de douceur ou des effets de brillance, par exemple.

Cependant, les polymères déposés sur les cheveux sont, en général, très vite éliminés lors des shampooings. Il est donc nécessaire de réappliquer du produit, au moins après chaque shampooing.

Il existe donc un besoin de réaliser des produits cosmétiques qui soient améliorés par rapport à ceux de l'art antérieur, et en particulier, et qui soient rémanents, face aux lavages répétés.

De manière surprenante et inattendue, la Demanderesse a découvert qu'il était possible d'atteindre les objectifs énumérés ci-dessus en sélectionnant les polymères introduits dans les produits cosmétiques, selon la nature des fonctions chimiques qu'ils portent, pour être appliquée sur les matières kératiniques, et en particulier sur les cheveux.

L'invention a donc pour premier objet une composition cosmétique comprenant, au moins un polymère PA et au moins un polymère PB, portant respectivement des fonctions chimiques issues de groupes A et B, identiques ou différentes, non photoactivables, caractérisée par le fait que :
(i) les fonctions chimiques issues des groupes A et B sont des fonctions chimiques complémentaires, et
(ii) les polymères PA et PB :
   (a) ne réagissent pas l'un avec l'autre pour former des liaisons covalentes, avant l'application de la composition cosmétique sur les matières kératiniques, notamment les cheveux,
   (b) sont aptes à réagir pour former des liaisons covalentes entre eux, après l'application de la composition cosmétique sur les matières kératiniques, notamment les cheveux .

Un second objet de la présente invention concerne un procédé cosmétique comprenant l'application de cette composition.

Un troisième objet de la présente invention concerne un procédé cosmétique comprenant l'application séparée ou étalée dans le temps sur des matières kératiniques, notamment sur des cheveux, de deux compositions contenant respectivement, pour l'une des deux, un ou plusieurs polymères de type PA, et pour l'autre, un ou plusieurs polymères de type PB, PA et PB étant des polymères à fonctions chimiques complémentaires.

Encore un autre objet de la présente invention concerne l'utilisation de ces procédés, pour réaliser un gainage sur les cheveux.

Conformément à l'invention, pour déterminer si des polymères PA et PB, portant respectivement des fonctions chimiques issues des groupes A et B, comprennent des fonctions chimiques complémentaires, on met en oeuvre le test décrit ci après, les opérations (1) à (4) étant conduites à la température ambiante:
(1) On applique 0,25 gramme d'une solution du polymère PA dans un solvant choisi parmi l'eau, l'éthanol, les esters, les cétones et de préférence l'eau, avec une teneur en poids du polymère par rapport au poids total de la solution, comprise entre 10 et 50 %, sur une lame de verre;
(2) On laisse le solvant du polymère PA s'évaporer, jusqu'à l'obtention d'un dépôt sec;
(3) On applique, sur la lame déjà recouverte par le polymère PA, 0,25 gramme d'une solution du polymère PB dans un solvant choisi parmi l'eau, l'éthanol, les esters, les cétones et de préférence l'eau, avec une teneur en poids du polymère PB par rapport au poids total de la solution, comprise entre 10 et 50 %;
(4) On laisse le solvant du polymère PB s'évaporer, jusqu'à l'obtention d'un dépôt sec;
(5) On place la lame de verre recouverte par les polymères PA et PB dans une enceinte à une température de 100 °C, et pendant une durée de 120 minutes ;
(6) Dans le cas où les solvants utilisés dans les points (1) et (3) sont différents, on répète une seconde fois les points (1) à (5);
(7) Le solide obtenu est entièrement immergé dans 10 grammes du solvant utilisé au point (1);
(8) Dans le cas où les solvants utilisés dans les points (1) et (3) sont différents, on immerge le solide obtenu à partir du point (6) dans 10 grammes du solvant utilisé au point (3) ;
(9) Les polymères PA et PB sont qualifiés de « *polymères comprenant des fonctions chimiques complémentaires* » si au moins 50 % en poids du solide ne se dissout pas au bout de 3 jours, à température ambiante et sans agitation, dans les solvants des points (7) et (8).

Dans les étapes (1) et (3), lorsque les solutions sont aqueuses, elles sont, de préférence, ajustées au pH d'utilisation des compositions de l'invention.

Sans être liée par la théorie, la Demanderesse pense que le dépôt solide insoluble peut traduire la formation de liaisons covalentes entre les polymères PA et PB (comme des réactions de substitution, d'addition sur des doubles ou triples liaisons - carbone-carbone, carbone-hétéroatome ou des réaction d'ouverture de cycles), des techniques de caractérisation connues de l'homme de l'art, telles que la spectrocopie Infra-rouge ou l'ESCA (XPS) pouvant, d'ailleurs, être utilisées pour évaluer, selon les cas, si et dans quelle mesure la formation de ces liaisons covalentes peut avoir eu lieu.

Les fonctions chimiques du groupe A sont choisies parmi les fonctions :
- Epoxyde,
- Aziridine,
- Vinyle et vinyle activée, en particulier, acrylonitrile, esters acrylique et métacrylique, acide et esters crotonique, acide et esters cinnamique, styrène et dérivés, butadiène, éthers de vinyle, vinylcétone, esters maléiques, vinyl sulfones, maléimides,
- Anhydride, chlorure d'acide et esters d'acide carboxylique,
- Aldéhydes,
- Acétals, hémi-acétals,
- Aminals, hémi aminals,
- Cétones, alpha-hydroxycétones, alpha-halocétones,
- Lactones, thiolactones,
- Isocyanate,
- Thiocyanate,
- Imines,
- Imides, en particulier, succinimide, glutimide,
- N hydroxysuccinimide esters,
- Imidates,
- thiosulfate,
- Oxazine et oxazoline,
- Oxazinium et oxazolinium,
- Halogénures d'alkyle en C1 à C30 ou d'aryle ou aralkyle en C6 à C30 de formule RX avec X = I, Br, Cl,
- Halogénure de cycle insaturé, carboné ou hétérocycle, notamment les chlorotriazine, chloropyrimidine, chloroquiinoxaline, chlorobenzotriazole,
- Halogénure de sulfonyle : RSO₂Cl ou F, R étant un alkyle en C1 à C30.
   Les fonctions chimiques du groupe B sont choisies parmi les fonctions XHₙ avec X= O, N, S, COO et n= 1 ou 2, notamment les alcool, amine, thiol et acide carboxylique.
   La présente invention exclut les polymères à fonctions chimiques photoactivables, c'est-à-dire les polymères comportant des fonctions chimiques, qui irradiées à une longueur d'onde comprise entre 200 et 800 nm, donnent naissance, en une ou plusieurs étapes, à la formation de nouvelles liaisons covalentes.
   Le produit conforme à l'invention peut donc se présenter sous la forme d'une composition comprenant l'association d'au moins deux polymères PA et PB, ayant des fonctions chimiques complémentaires, dans la mesure où le milieu cosmétiquement acceptable est choisi de telle sorte que ces deux polymères restent inertes l'un vis-à-vis de l'autre dans la composition, mais réagissent, en formant des liaisons covalentes, après application de la composition sur les cheveux.
   Le produit conforme à l'invention peut, également, se présenter sous la forme d'une association de compositions, et notamment un kit comprenant au moins deux compartiments, le premier contenant une composition comprenant au moins un polymère PA et le second, au moins un polymère PB, PA et PB, ayant des fonctions chimiques complémentaires, dans un milieu cosmétiquement acceptable, identique ou différent.
   Lorsque le produit conforme à l'invention forme une association de compositions, notamment un kit, les compositions élémentaires sont utilisées de façon séparée ou étalée dans le temps, une ou plusieurs applications de ces dernières pouvant avoir lieu.
   Les groupements chimiques A peuvent réagir avec les groupements B, soit spontanément, soit par une activation par la température, le pH, un co-réactif ou un catalyseur chimique ou biochimique, comme une enzyme.
   De préférence, on choisit, comme fonctions chimiques du groupe A, les groupes anhydride, époxydes, chlorotriazine, aldéhydes, thiosulfates ainsi que, pour les fonctions chimiques du groupe B, les fonctions hydroxy, amines primaires et secondaires, les thiols, les acides carboxyliques.
   Avantageusement, chaque polymère de type PA et PB possède au moins deux fonctions chimiques de type A ou B identiques.
   Les polymères selon l'invention peuvent contenir des fonctions chimiques autres que celles des groupes A et B.
   De préférence, les polymères portant des fonctions chimiques anhydride ne comportent pas de fonctions acides carboxyliques.
   Par « polymère », on entend un composé comportant au moins 5 motifs de répétition enchaînés par des liaisons covalentes.
   Le polymère peut être synthétisé
- par des réactions radicalaires (polyacrylates, polyméthacrylates, polyvinyles...),
- par des réactions de condensation (polyesters, polyéthers, polyamides, polyuréthanes, polydiméthylsiloxanes, polypeptides...)
- par des réactions d'ouverture de cycle (polyesters ...).

Il peut être d'origine naturelle, modifié chimiquement ou non, comme par exemple les polysaccharides (cellulose, dextrane, chitosane, guar et leurs dérivés hydroxyalkylés, carboxyméthylés, aminés ou thiolés, ou leurs dérivés à fonction aldéhyde ou époxy).

Les polymères peuvent se présenter sous tout type de topologie : chaîne linéaire, ramifiée, en étoile ou hyperbranchée (comme les dendrimères par exemple), chaînes séquencées, statistiques ou alternées.

Les groupements chimiques peuvent être naturellement présents sur la chaîne polymérique, en bout de chaîne, greffés le long de la chaîne principale ou des chaînes secondaires, sur les branches des polymères en étoile ou hyperbranchés.

Chaque polymère possède au moins un groupement chimique de type A ou B.

De préférence, chaque polymère possède au moins deux groupements chimiques identiques (A,A ou B,B), afin de se lier avec au moins deux autres polymères.

On préfère les associations de polymères suivants :
- les dendrimères à terminaisons OH, ou NH₂ ou SH ou COOH de une ou plusieurs générations en association avec les polymères contenant le motif anhydride, notamment maléique, ou les polymères contenant le groupe époxyde et/ou aldéhyde,
- les polymères synthétiques à fonction hydroxyles, tels les alcools polyvinyliques, en association avec les polymères contenant le motif anhydride, notamment maléique ou les polymères contenant le groupe époxyde,
- les polyéthylèneimine en association avec les polymères contenant le motif anhydride, notamment maléique ou les polymères contenant le groupe époxyde et/ou aldéhyde,
- les polyéthylèneimine thiols, obtenus par réaction de polyéthylèneimines sur la γ-butyrolactone, tels que ceux décrits dans le brevet L'OREAL FR2772770, en association avec les polymères contenant le motif anhydride, notamment maléique ou les polymères contenant le groupe époxyde et/ou aldéhyde,
- les polyacides aminés présentant des groupes OH, ou NH₂ ou SH ou COOH libres, par exemple la polylysine en association avec les polymères contenant le motif anhydride, notamment maléique ou les polymères contenant le groupe époxyde et/ou aldéhyde,
- les polysaccharides naturels ou modifiés présentant des fonctions OH, ou NH₂ ou SH ou COOH en association avec les polymères contenant le motif anhydride, notamment maléique ou les polymères contenant le groupe époxyde et/ou aldéhyde,
- les polysaccharides naturels ou modifiés présentant des fonctions aldéhydes en association avec les polymères synthétiques ou naturels à fonctions OH ou NH₂ ou SH ou COOH comme par exemple les polyéthylèneimine, la polylysine, le chitosane et ses dérivés, tels que les carboxyméthyl-chitosanes ou les amino-dextranes,
- les polysaccharides naturels ou modifiés présentant des fonctions époxy en association avec les polymères synthétiques ou naturels à fonctions OH ou NH₂ ou SH ou COOH comme par exemple la polyéthylèneimine, la polylysine, le chitosan et ses dérivés, tels que les carboxyméthyl-chitosanes ou les amino-dextranes,
- les polymères naturels ou synthétiques à fonctions acide carboxylique en association avec les polymères synthétiques ou naturels à fonctions OH, NH₂ ou SH, en présence d'un carbodiimide, ou d'un acide, d'une base ou d'une enzyme, comme les estérase, lipase, protéase.

### Par exemple et sans limitation, on cite les polymères réactifs suivants :

### Polymères à fonctions du groupe A

- Copolymère méthyl-vinyl éther/ anhydride maléique, en particulier commercialisé par exemple par ISP sous le nom de Gantrez,
- PolyMethacrylate de glycidyle, en particulier commercialisé par Polysciences,
- Polydimethylsiloxane de glycidyle, en particulier commercialisé par Shinetsu (référence X-2Z-173 FX ou DX),
- Polyamidoamine époxy, par exemple commercialisé par Hercules sous le nom de Delsette 101, le Kymène 450 de chez Hercules,
- Epoxy-dextrane,
- Polysaccharides polyaldéhydes obtenus par oxydation de polysaccharides par NaIO4 (méthodes connues, Bioconjugate Techniques ; Hermanson GT, Academic Press, 1996),

### Polymères à fonctions du groupe B

- Dendrimère PAMAM, en particulier commercialisé par Dendritech, DSM, Sigma-Aldrich (STARBURST, PAMAM DENDRIMER, G(2, O) de chez DENDRITECH),
- Dendrimère à fonctions hydroxy, en particulier commercialisé par Perstorp, DSM, (exemple : HBP TMP core 2 Generation PERSTORP)
- PEI (polyéthylène-imine), en particulier commercialisé par BASF, sous le nom de Lupasol,
- PEI-Thiol,
- Polylysine, en particulier commercialisé par Chisso,
- HP cellulose, tel que KLUCELEF de chez AQUALON),
- Amino-dextrane, par exemple commercialisé par Carbomer,
- Amino-cellulose, par exemple ceux décrits dans WO01/25283 de BASF,
- PVA (polyvinylacétal), par exemple AIRVOL 540 de chez AIRPRODUCTS CHEMICAL),
- Amino PVA, par exemple commercialisé par Carbomer,
- Chitosane,
- CM ou HP Dextrane, par exemple commercialisé par Fluka,
- CM ou HP Chitosane, par exemple commercialisé par Fluka,

Toutes les associations d'au moins un polymère du groupe A et d'au moins un polymère du groupé B sont citées, en exemple, sans limitation.

Les polymères PA et PB, portant respectivement des fonctions chimiques issues de groupes A et B, peuvent réagir entre eux pour former des liaisons covalentes, par exemple, selon les protocoles suivants.

### Variantes des procédés d'application

a) Dans le cas où la réaction entre les différents polymères a lieu spontanément à température ambiante mais leur mélange en solution diluée est stable, on peut appliquer directement sur la matière une solution contenant les polymères dans un solvant volatile cosmétiquement acceptable et au cours de l'évaporation du solvant, la réaction de réticulation a lieu. Le dépôt de polymères devient insoluble et reste sur la matière.
b) Dans le cas où la réaction entre les différents polymères nécessite une activation, on peut appliquer le mélange des polymères sur la matière et par augmentation de la température ou ajout d'un agent modificateur de pH, ou ajout d'un co-réactif ou d'un catalyseur, on provoque la réticulation du dépôt.
c) Avantageusement, dans le cas où l'un des polymères présente une affinité particulière pour la matière, on dépose d'abord sur la matière ce premier polymère via un solvant volatile cosmétiquement acceptable, puis on dépose le ou les polymères susceptibles de réagir avec le premier, via un solvant volatile cosmétiquement acceptable. La réaction chimique peut alors avoir lieu spontanément au cours du séchage ou être déclenchée par un apport de chaleur, une modification de pH, un ajout de co-réactif ou de catalyseur. Dans ce cas, le produit conforme à l'invention est généralement un kit. Le dépôt réticulé ainsi formé présente l'avantage d'avoir une faible solubilité attendue. En outre, il possède une bonne affinité pour la surface de la matière, ce qui garantit une meilleure rémanence de l'ensemble du dépôt.

Ce type de dépôt en couches peut aussi être avantageux pour conserver les propriétés cosmétiques ou optiques du polymère qui constitue la partie supérieure du dépôt.

Selon les mêmes procédés, il est possible de réaliser des superpositions multiples de couches de polymères qui réticulent entre eux pour atteindre le type de dépôt souhaité (en termes de nature chimique, résistance mécanique, épaisseur, aspect, toucher...).

Le milieu cosmétiquement acceptable, véhiculant le ou les polymères de type A et/ou les polymères de type B, est choisi de telle sorte que les polymères à fonctions chimiques complémentaires soient aptes à réagir l'un avec l'autre pour former des liaisons covalentes après l'application de la composition cosmétique sur les cheveux. De préférence, le milieu cosmétiquement acceptable comprend un activateur approprié choisi parmi les agents modificateur de pH, les co-réactifs et les catalyseurs.

Avantageusement, on choisit :
- comme modificateur de pH : acides ou bases, de nature minérale ou organique ;
- comme co-réactif : carbodiimide, oxydants ou réducteurs ;
- comme catalyseur : enzymes.
   Les procédés conformes à l'invention comprennent l'application sur les cheveux de compositions cosmétiques ci-dessus.
   Avantageusement, il comprend les opérations supplémentaires consistant à provoquer une modification de pH et/ou, une élévation de température et/ou procéder à l'ajout d'un ou plusieurs additifs et/ou rincer.
   Selon un mode de réalisation de l'invention, on applique une composition de soin, de coloration, de déformation permanente, de maquillage du cheveu, de fixation et/ou de maintien de la coiffure, avant d'appliquer un produit conforme à l'invention.
   Le ou les polymères à fonctions chimiques complémentaires sont, de préférence, présents à des concentrations comprises entre 0,05 et 50 % en poids, plus préférentiellement comprises entre 0,1 et 20 % en poids, et plus préférentiellement entre 0,25 et 10 % en poids par rapport au poids total de la ou des composition(s) selon l'invention.
   La ou les compositions contenant le ou les polymères à fonctions complémentaires selon l'invention contiennent avantageusement, en outre, des additifs cosmétiques conventionnels choisis parmi les polymères fixants, les épaississants, les tensioactifs anioniques, non ioniques, cationiques ou amphotères, les parfums, les conservateurs, les filtres solaires, les protéines, les vitamines, les provitamines, les polymères non fixants anioniques, non ioniques, cationiques ou amphotères, les huiles minérales, végétales ou synthétiques, les céramides, les pseudocéramides, les silicones volatiles ou non, linéaires ou cycliques, modifiées ou non, des agents régulateurs de pH, des oxydants, des réducteurs, des inhibiteurs, des catalyseurs et tout autre additif classiquement utilisé dans les compositions cosmétiques.
   Le milieu cosmétiquement acceptable est, de préférence, constitué par de l'eau et/ou un ou plusieurs solvants cosmétiquement acceptables tels que des alcools, les esters, les cétones ou les silicones volatiles cycliques ou des mélanges eau-solvant(s), ces solvants étant de préférence des alcools en C₁-C₄.
   Lorsque la ou les compositions selon l'invention est (sont) conditionnée(s) dans un dispositif aérosol, il comprend au moins un agent propulseur, qui peut être choisi parmi les hydrocarbures volatils tels que le n-butane, le propane, l'isobutane, le pentane, les hydrocarbures halogénés et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyl éther.
   Avantageusement, l'agent propulseur est présent à une concentration comprise entre 5 et 90 % en poids par rapport au poids total de la composition dans le dispositif aérosol et, plus particulièrement, à une concentration comprise entre 10 et 60 %.
   Les produits conformes à l'invention peuvent être appliqués sur des cheveux secs ou humides.
   L'invention va être plus complètement illustrée à l'aide de l'exemple non limitatif suivant.

### Exemples :

Composition A : Dendrimères Starbust PAMAM, génération 2, commercialisée en solution aqueuse par Dendritech
Composition B : Solution aqueuse à 5% de Gantrez S-97BF, commercialisé par ISP
Composition C : mélange des deux réactifs
   - composition A : 50g
   - composition B : 50g
Composition D : mélange des deux réactifs avec n actif cosmétique (panthénol)
   - composition A : 45g
   - panthénol : 1g
   - eau qsp 50g
Actif cosmétique : panthénol

### Protocole en applications successives

On travaille sur cheveux naturels, avec une mèche de 2.7g.
0.3 g de composition A est appliquée sur cheveux propres et secs, suivie de l'application de 0.25g de composition B. Le dépôt est séché au sèche-cheveux pendant 45 minutes puis la mèche est laissée pendant deux heures à 100°C.

Le dépôt ainsi réalisé sur la matière est rémanent à 10 shampooings.

Juste après application et traitement thermique, on observe un gainage épais et couvrant qui recouvre totalement les écailles du cheveu. Après 10 shampooings, le gainage semble s'être affiné, notamment en pointes mais recouvre encore nettement le cheveu.

### Protocole en Mono-applications

On travaille sur cheveux naturels, avec une mèche de 2.7g.
0.5 g de composition C est appliqué sur cheveux propres et secs. Le dépôt est séché au sèche-cheveux pendant 45 minutes puis la mèche est laissée pendant deux heures à 100°C.
Le dépôt ainsi réalisé sur la matière est rémanent à 10 shampooings.

### Inclusion d'un actif cosmétique dans le dépôt réticulé

On travaille sur cheveux naturels, avec une mèche de 2.7g.
0.3 g de la composition D est appliquée sur cheveux propres et secs, suivie de l'application de 0.25g de la composition B. Le dépôt est séché au sèche-cheveux pendant 45 minutes puis la mèche est laissée pendant deux heures à 100°C.
Le dépôt ainsi réalisé sur la matière est rémanent à 10 shampooings et contient du panthénol.

## Revendications

1. Composition cosmétique comprenant au moins un polymère PA et au moins un polymère PB, portant respectivement des fonctions chimiques issues de groupes A et B, identiques ou différentes, non photoactivables, **caractérisée par le fait que** :
(i) les fonctions chimiques issues des groupes A et B sont des fonctions chimiques complémentaires, et
(ii) les polymères PA et PB :
(a) ne réagissent pas l'un avec l'autre pour former des liaisons covalentes, avant l'application de la composition cosmétique sur les matières kératiniques, notamment les cheveux,
(b) sont aptes à réagir pour former des liaisons covalentes entre eux, après l'application de la composition cosmétique sur les matières kératiniques, notamment les cheveux, les fonctions chimiques du groupe A étant choisies parmi les fonctions :
- Epoxyde,
- Aziridine,
- Vinyle et vinyle activée, en particulier, acrylonitrile, esters acrylique et métacrylique, acide et esters crotonique, acide et esters cinnamique, styrène et dérivés, butadiène, éthers de vinyle, vinylcétone, esters maléiques, vinyl sulfones, maléimides,
- Anhydride, chlorure d'acide et esters d'acide carboxylique,
- Aldéhydes,
- Acétals, hémi-acétals,
- Aminals, hémi aminals,
- Cétones, alpha-hydroxycétones, alpha-halocétones,
- Lactones, thiolactones,
- Isocyanate,
- Thiocyanate,
- Imines,
- Imides, en particulier, succinimide, glutimide,
- N hydroxysuccinimide esters,
- Imidates,
- thiosulfate,
- Oxazine et oxazoline,
- Oxazinium et oxazolinium,
- Halogénures d'alkyle en C1 à C30 ou d'aryle ou aralkyle en C6 à C30 de formule RX avec X = I, Br, Cl,
- Halogénure de cycle insaturé, carboné ou hétérocycle, notamment les chlorotriazine, chloropyrimidine, chloroquiinoxaline, chlorobenzotriazole,
- Halogénure de sulfonyle : RSO₂Cl ou F, R étant un alkyle en C1 à C30 ;
les fonctions chimiques du groupe B étant choisies parmi les fonctions XHₙ avec X= O, N, S, COO et n= 1 ou 2, notamment les alcool, amine, thiol et acide carboxylique.

2. Composition selon la revendication 1, **caractérisée par le fait que** les fonctions chimiques du groupe A sont choisis parmi les fonctions anhydride, époxydes, chlorotriazine, aldéhydes, thiosulfates.

3. Composition selon la revendication 1, **caractérisée par le fait que** les fonctions chimiques du groupe B sont choisies parmi les fonctions hydroxy, amines primaires et secondaires, les thiols, les acides carboxyliques.

4. Composition selon l'une quelconque des revendications précédentes, **caractérisée par le fait que** chaque polymère de type PA et PB possède au moins deux fonctions chimiques de type A ou B identiques.

5. Composition selon la revendication 1, **caractérisée par le fait que** les polymères PA et PB sont choisies parmi les associations suivantes:
- les dendrimères à terminaisons OH, ou NH₂ ou SH ou COOH de une ou plusieurs générations en association avec les polymères contenant le motif anhydride, notamment maléique, ou les polymères contenant le groupe époxyde et/ou aldéhyde,
- les polymères synthétiques à fonction hydroxyles, tels les alcools polyvinyliques, en association avec les polymères contenant le motif anhydride, notamment maléique ou les polymères contenant le groupe époxyde,
- les polyéthylèneimine en association avec les polymères contenant le motif anhydride, notamment maléique ou les polymères contenant le groupe époxyde et/ou aldéhyde,
- les polyéthylèneimine thiols, obtenus par réaction de polyéthylèneimines sur la γ-butyrolactone, en association avec les polymères contenant le motif anhydride, notamment maléique ou les polymères contenant le groupe époxyde et/ou aldéhyde,
- les acides aminés présentant des groupes OH, ou NH₂ ou SH ou COOH libres, par exemple la polylysine en association avec les polymères contenant le motif anhydride, notamment maléique ou les polymères contenant le groupe époxyde et/ou aldéhyde,
- les polysaccharides naturels ou modifiés présentant des fonctions OH, ou NH₂ ou SH ou COOH en association avec les polymères contenant le motif anhydride, notamment maléique ou les polymères contenant le groupe époxyde et/ou aldéhyde,
- les polysaccharides naturels ou modifiés présentant des fonctions aldéhydes en association avec les polymères synthétiques ou naturels à fonctions OH ou NH₂ ou SH ou COOH,
- les polysaccharides naturels ou modifiés présentant des fonctions époxy en association avec les polymères synthétiques ou naturels à fonctions OH ou NH₂ ou SH ou COOH,
- les polymères naturels ou synthétiques à fonctions acide carboxylique en association avec les polymères synthétiques ou naturels à fonctions OH, NH₂ ou SH, en présence d'un carbodiimide, ou d'un acide, d'une base ou d'une enzyme, comme les estérase, lipase, protéase.

6. Composition selon la revendication 1, **caractérisée par le fait que** le polymère à fonctions chimiques du groupe A est choisi parmi ceux énumérés ci-après :
- Copolymère méthyl-vinyl éther/ anhydride maléique,
- PolyMethacrylate de glycidyle,
- Polydiméthylsiloxane de glycidyle,
- Polyamidoamine époxy,
- Epoxy-dextrane,
- Polysaccharides polyaldéhydes obtenus par oxydation de polysaccharides par NaIO4.

7. Composition selon la revendication 1, **caractérisée par le fait que** le polymère à fonctions chimiques du groupe B est choisi parmi ceux énumérés ci-après :
- Dendrimère PAMAM,
- Dendrimère à fonctions hydroxy,
- PEI (polyéthylène-imine),
- PEI-Thiol,
- Polylysine,
- HP cellulose,
- Amino-dextrane,
- Amino-cellulose,
- PVA (polyvinylacétal),
- Amino PVA,
- Chitosane,
- CM ou HP Dextrane, et
- CM ou HP Chitosane.

8. Composition selon l'une quelconque des revendications précédentes, **caractérisée par le fait que** le ou les polymères à fonctions chimiques complémentaires sont présents à des concentrations comprises entre 0,05 et 50 % en poids, plus préférentiellement comprises entre 0,1 et 20 % en poids, et plus préférentiellement entre 0,25 et 10 % en poids par rapport au poids total de la ou des composition(s).

9. Composition selon l'une quelconque des revendications précédentes, **caractérisée par le fait qu'**elle contient, en outre, des additifs cosmétiques conventionnels choisis parmi les polymères fixants, les épaississants, les tensioactifs anioniques, non ioniques, cationiques ou amphotères, les parfums, les conservateurs, les filtres solaires, les protéines, les vitamines, les provitamines, les polymères non fixants anioniques, non ioniques, cationiques ou amphotères, les huiles minérales, végétales ou synthétiques, les céramides, les pseudocéramides, les silicones volatiles ou non, linéaires ou cycliques, modifiées ou non, des agents régulateurs de pH, des oxydants, des réducteurs, des inhibiteurs, des catalyseurs et tout autre additif classiquement utilisé dans les compositions cosmétiques.

10. Composition selon l'une quelconque des revendications précédentes, **caractérisée par le fait que** le milieu cosmétiquement acceptable est constitué par de l'eau et/ou un ou plusieurs solvants cosmétiquement acceptables tels que des alcools, les esters, les cétones ou les silicones volatiles cycliques ou des mélanges eau-solvant(s).

11. Procédé cosmétique capillaire, **caractérisé par le fait qu'**il comprend l'application d'une composition selon l'une quelconque des revendications 1 à 10.

12. Procédé selon la revendication 11, **caractérisé par le fait qu'**on utilise une activation extérieure pour activer la composition, de préférence une modification de la température.

13. Procédé selon l'une quelconque des revendications 11 ou 12, **caractérisé par le fait qu'**on applique une composition de soin, de coloration, de déformation permanente, de maquillage du cheveu, de fixation et/ou de maintien de la coiffure, avant d'appliquer un produit conforme à l'invention.

14. Kit comprenant au moins deux compartiments, le premier contenant une composition comprenant au moins un polymère PA et le second, au moins un polymère PB, PA et PB, ayant des fonctions chimiques complémentaires, dans un milieu cosmétiquement acceptable, identique ou différent.

15. Procédé cosmétique comprenant l'application séparée ou étalée dans le temps sur des matières kératiniques, notamment sur des cheveux, de deux compositions contenant respectivement, pour l'une des deux, un ou plusieurs polymères de type PA, et pour l'autre, un ou plusieurs polymères de type PB, PA et PB étant des polymères à fonctions chimiques complémentaires.

16. Utilisation d'une composition selon l'une quelconque des revendications 1 à 10 ou d'un kit selon la revendication 14 ou d'un procédé selon les revendications 11 à 13 ou 15, pour former un gainage sur les cheveux.
